**Europäisches Patentamt**

**European Patent Office**

**Office européen des brevets**

(19)

(11) Veröffentlichungsnummer: **0 185 689**
**B1**

(12) # EUROPÄISCHE PATENTSCHRIFT

(45) Veröffentlichungstag der Patentschrift:
**03.05.89**

(21) Anmeldenummer: **85902465.5**

(22) Anmeldetag: **22.05.85**

(86) Internationale Anmeldenummer:
**PCT/DE 85/00172**

(87) Internationale Veröffentlichungsnummer:
**WO 86/00021 (03.01.86 Gazette 86/1)**

(51) Int. Cl.⁴: **A 61 L 25/00**, A 61 K 6/06,
A 61 K 49/04

(54) **VERBUNDWERKSTOFF AUF KUNSTSTOFFBASIS FÜR PROTHETISCHE ZWECKE.**

(30) Priorität: **07.06.84 DE 3421157**

(43) Veröffentlichungstag der Anmeldung:
**02.07.86 Patentblatt 86/27**

(45) Bekanntmachung des Hinweises auf die Patenterteilung:
**03.05.89 Patentblatt 89/18**

(84) Benannte Vertragsstaaten:
**AT BE CH DE FR GB IT LI**

(56) Entgegenhaltungen:
**EP-A- 0 102 199**
**FR-A- 2 370 468**
**US-A- 4 503 169**

(73) Patentinhaber: **Wild Leitz GmbH,
Ernst-Leitz-Strasse 30 Postfach 20 20,
D-6330 Wetzlar 1 (DE)**
Patentinhaber: **Kulzer & Co. GmbH,
Philipp-Reis-Strasse 8, D-6393 Wehrheim (TS.)1 (DE)**

(72) Erfinder: **FRANEK, Henning, Mühlenkopfstr. 5,
D-6333 Braunfels-Tiefenbach (DE)**
Erfinder: **BRÖMER, Heinz, Hundsrück 7,
D-6330 Wetzlar 26 (DE)**
Erfinder: **DEUTSCHER, Klaus, Lerchenweg 20,
D-6330 Wetzlar 1 (DE)**
Erfinder: **SCHAEFER, Roland, Merianweg 5,
D-6382 Friedrichsdorf 2 (DE)**

## Beschreibung

Die Anmeldung betrifft einen Verbundwerkstoff auf Kunststoffbasis für prothetische Zwecke, der Füllstoffe hoher Röntgenabsorption auf der Basis glasiger bzw. glaskeramischer Systeme aufweist.

Verbundwerkstoffe (Kompositmaterialien bzw. Komposite) auf Kunststoffbasis haben in jüngster Vergangenheit eine immer größere Bedeutung erlangt. Zusätze von anorganischen Füllstoffen zu polymerisierbaren organischen Bindemitteln reduzieren nicht nur die Schrumpfung bei der Polymerisation, sondern verbessern auch die mechanischen Festigkeitswerte, wie z. B. Druckfestigkeit, Biegefestigkeit und Elastizitätsmodul. Gleichzeitig vermindert sich auch der thermische Ausdehnungskoeffizient des Komposits gegenüber dem der reinen Kunststoffe.

Durch Zugabe von anorganischen Füllstoffen zu organischen Bindemitteln können die speziellen Eigenschaften der jeweils verwendeten Füllstoffe in das Gesamt-Verbundsystem eingebracht werden. So konnte z. B. bei dem für prothetische Zwecke eingesetzten Polymethylmethacrylat (PMMA) die Eigenschaft der Bildung eines bindegewebsfreien Verbundes zwischen bioaktivem Implantatmaterial und Knochen auf dieses System dadurch übertragen werden, daß bioaktive Glaskeramik in Granulatform in das PMMA eingebracht wird, vgl. hierzu die DE-PS 2501683.

Aus der US-PS 3066112 war es darüber hinaus bekannt, bei Dentalwerkstoffen auf Kunststoffbasis das Methylmethacrylat durch das Reaktionsprodukt aus Glycidylmethacrylat und Bisphenol A, nämlich: Bis-[4-(2-hydroxy-3-methacryloyloxy-propoxy)-phenyl]-dimethylmethan (auch Bis-GMA genannt), zu ersetzen und als anorganischen Füllstoff mit einem Vinylsilan behandeltes Quarzpulver zuzugeben.

Der anorganische Füllstoff kann bei geeigneter chemischer Zusammensetzung auch dazu dienen, den von Natur aus gegenüber Röntgenstrahlen durchlässigen Kunststoffen die Eigenschaft der Röntgenabsorption zu verleihen, so daß sie auf dem Röntgenfilm sichtbar werden. Aus der US-PS 3539526 ist bekannt, als röntgenopaken Zusatz in Zahnfüllmaterial ein aus $SiO_2$, $BaF_2$, $Al_2O_3$ und $B_2O_3$ hergestelltes feinteiliges Glas zu verwenden. Diese Füllstoffe enthalten oft einen hohen Bariumanteil; ihre Röntgenabsorption ist jedoch nicht hoch genug, um sie für Kompositmaterialien mit Erfolg einzusetzen, die im Seitenzahnbereich Verwendung finden. Bekannte Komposite mit bariumhaltigen Füllstoffen erreichen eine Röntgenopazität von ca. 200%.

Es sind außerdem auch röntgenopake Gläser bekannt, deren Röntgenstrahlen-Absorption durch Zusatz von Verbindungen des Strontiums und Lanthans bzw. des Strontiums und des Wolframs bewirkt wird, vgl. die DE-OS 2458380. Allerdings ist die Absorption der Röntgenstrahlen auch dieser Gläser nicht hoch genug, um mit ihrer Hilfe ein Kompositmaterial genügend hoher Röntgenopazität für den Seitenzahnbereich herzustellen.

Aufgabe der vorliegenden Erfindung ist es daher, einen Verbundwerkstoff auf Kunststoffbasis für prothetische Zwecke mit einer möglichst hohen Röntgenopazität und hohen mechanischen Werten (Druck- und Biegefestigkeit) bereitzustellen. Der Einsatz des Verbundwerkstoffes für medizinische Zwecke stellt zusätzliche Anforderungen an den Füllstoff, da der Ausschluß jedweder toxischer Bestandteile sowie eine ausreichende chemische Stabilität gegenüber den Körperflüssigkeiten im allgemeinen und dem Mundmilieu im besonderen zu fordern ist. Bei Verwendung als Dentalmaterial – z. B. für künstliche Zahnkronen oder Brücken – kommt die Forderung einer guten Transluzenz und einer guten Polierbarkeit hinzu. Für orthopädische Applikationen – zum Beispiel als Knochenzement – muß die niedrige Viskosität der organischen Bindemittel trotz Zusatz der Füllstoffe erhalten bleiben, um eine gute Fließeigenschaft und eine optimale Verankerungsfähigkeit auch in spongiösem Knochenmaterial zu gewährleisten.

Die Aufgabe wird erfindungsgemäß durch einen röntgenopaken Füllstoff enthaltenden Verbundwerkstoff auf Kunststoffbasis für prothetische Zwecke gelöst, wobei der röntgenopake Füllstoff Verbindungen der Elemente Gadolinium (Gd), Strontium (Sr) und gegebenenfalls Lanthan (La) als röntgenabsorptionsverursachende Komponenten-Kombination enthält. Vorteilhafterweise enthält der röntgenopake Füllstoff die Oxid-Gruppe: Gadoliniumoxid ($Gd_2O_3$) + Strontiumoxid (SrO) bzw. Gadoliniumoxid ($Gd_2O_3$) + Strontiumoxid (SrO) + Lanthanoxid ($La_2O_3$). Der Anteil des röntgenopaken Füllstoffs am Gesamt-Verbundwerkstoff kann zwischen 20 und 90 Gew.-% – vorzugsweise 40 bis 85 Gew.-% – betragen.

Nach einer weiteren Ausgestaltung der vorliegenden Erfindung besteht der röntgenopake Füllstoff aus 1 bis 31 Gew.-% Gadoliniumoxid ($Gd_2O_3$), 7 bis 32 Gew.-% Strontiumoxid (SrO), 0 bis 18 Gew.-% Lanthanoxid ($La_2O_3$), wobei die Summe der Oxide $Gd_2O_3$ + SrO + $La_2O_3$ zwischen 24 und 40 Gew.-% beträgt; aus 14 bis 62 Gew.-% Siliciumdioxid ($SiO_2$), 0 bis 30 Gew.-% Bortrioxid ($B_2O_3$), 1 bis 33 Gew.-% Aluminiumoxid ($Al_2O_3$), wobei die Summe der Oxide $SiO_2$ + $B_2O_3$ + $Al_2O_3$ zwischen 42 und 75 Gew.-% beträgt; sowie aus 0 bis 16 Gew.-% Natriumoxid ($Na_2O$). Es ist möglich, daß der SrO-Anteil entweder bis zu 18 Gew.-% durch Calciumoxid (CaO) oder bis zu 5 Gew.-% durch Magnesiumoxid (MgO) ersetzt wird. Auch liegt es im Rahmen der vorliegenden Erfindung, den Anteil an $Gd_2O_3$ und/oder $La_2O_3$ und/oder SrO bis zu 5 Gew.-% durch Zirkondioxid ($ZrO_2$) zu ersetzen. Schliesslich kann die Rezeptur auch in der Weise abgewandelt werden, daß der Anteil an $Gd_2O_3$ und/oder $La_2O_3$ und/oder SrO bis zu 5 Gew.-% durch mindestens eines der Oxide: Yttriumoxid ($Y_2O_3$), Titandioxid ($TiO_2$), Tantaloxid ($Ta_2O_5$) und Nioboxid ($Nb_2O_5$) ersetzt wird. Eine zweckmäßige Zusammensetzung des röntgenopaken Füllstoffs besteht aus: 1,0 bis 26,9 Gew.-% $Gd_2O_3$, 10,9 bis 28,8 Gew.-% SrO, 0 bis 12,0 Gew.-% $La_2O_3$, 16,1 bis 58,0 Gew.-% $SiO_2$, 0 bis 26,3 Gew.-% $B_2O_3$, 2,0

bis 32,0 Gew.-% $Al_2O_3$, 0 bis 16,0 Gew.-% $Na_2O$, 0 bis 15,0 Gew.-% CaO, 0 bis 4,0 Gew.-% MgO, 0 bis 5,0 Gew.-% $ZrO_2$, 0 bis 5,0 Gew.-% $Y_2O_3$, 0 bis 4,0 Gew.-%$TiO_2$, 0 bis 5,0 Gew.-% $Ta_2O_5$ und 0 bis 5,0 Gew.-% $Nb_2O_5$. Nach einer besonderen Ausführungsform der vorliegenden Erfindung besteht der röntgenopake Füllstoff aus 2,0 bis 10,0 Gew.-% $Gd_2O_3$, 15,0 bis 25,0 Gew.-% SrO, 1,0 bis 10,0 Gew.-% $La_2O_3$, 50,0 bis 60,0 Gew.-% $SiO_2$, 2,0 bis 6,0 Gew.-% $B_2O_3$, 1,0 bis 3,0 Gew.-% $Al_2O_3$, 2,0 bis 8,0 Gew.-% $Na_2O$ und 1,0 bis 5,0 Gew.-% CaO. Der röntgenopake Füllstoff kann als Glas mit einer Brechzahl $n_e$ zwischen 1,55 und 1,62 und/oder als Glaskeramik und/oder als Sinterprodukt und/oder in Form eines Gemisches (Gemenge) aus seinen Ausgangskomponenten vorliegen.

Der erfindungsgemäße Verbundwerkstoff kann zusätzlich Pigmente mit einer Korngrösse kleiner als 2 µm enthalten. Es ist zweckmäßig, daß der röntgenopake Füllstoff in Form von silanisierten Partikeln vorliegt. Die Partikelgröße liegt im Bereich zwischen 0,5 und 50 µm. Es hat sich als vorteilhaft erwiesen, daß der Verbundwerkstoff zusätzlich bis zu 30 Gew.-% hochdisperses Siliciumdioxid ($SiO_2$) und/oder Aluminiumoxid ($Al_2O_3$) enthält. Ein Vorprodukt für den Verbundwerkstoff kann als organisches Bindemittel mindestens einen Acrylsäureester und/oder einen Methacrylsäureester sowie den röntgenopaken Füllstoff enthalten. Zusätzlich kann dem Vorprodukt ein Polymerisationskatalysator, insbesondere ein Photopolymerisationskatalysator, zugegeben werden. Mit Vorteil läßt sich dieses Vorprodukt bzw. dieser Verbundwerkstoff als Dentalmaterial, insbesondere als Zahnfüllmaterial, verwenden. Ebenso ist eine Verwendung des Vorprodukts als Knochenzement möglich.

Die Beachtung des erfindungsgemäßen Brechzahlintervalls des glasigen Füllstoffs ist für eine Applikation im Dentalbereich von großer Bedeutung, um eine ästhetische Wirkung zu erzielen. Die durch Vermischung des röntgenopaken Füllstoffs mit den organischen Bindemitteln erhaltenen pastenförmigen Vorprodukte besitzen eine gute Plastizität. Sie sind nicht klebrig und lassen sich daher gut verarbeiten. Dabei hat es sich als besonders vorteilhaft erwiesen, den anorganischen Füllstoff durch Behandlung mit Kupplungsreagenzien, beispielsweise Vinylsilanen – insbesondere γ-Methacryloyloxypropyltrimethoxysilan oder Vinyltriäthoxysilan – wie aus der DE-PS 1937871 bekannt, zu silanisieren.

Als polymerisierbare Bindemittel können alle für diese Zwecke bekannten Verbindungen eingesetzt werden. Für medizinische Anwendungen sind Acryl- und Methacrylsäureester ein- und mehrwertiger Alkohole, z.B. das bekannte Bis-GMA und die aus der DE-OS 2312559 bekannten sogenannten Urethanacrylate und -methacrylate, bevorzugt.

Die Polymerisation bzw. das Aushärten des erfindungsgemäßen Vorprodukts kann bei Raumtemperatur (Kaltpolymerisation), in der Wärme (Heißpolymerisation) oder auch durch Bestrahlen mit sichtbarem Licht oder UV-Licht (Photopolymerisation) unter Zusatz von geeigneten Katalysatoren erfolgen.

Solche Katalysatoren sind z.B. für die Kaltpolymerisation Dibenzoylperoxid/N,N-Dimethyl-p-toluidin, für die Heißpolymerisation Dibenzoylperoxid und für die Photopolymerisation Benzil/N,N-Dimethylaminoäthylmethacrylat bzw. Benzoinmethyläther.

Mit der vorgeschlagenen Füllstoff-Zusammensetzung wird ein deutlich verbessertes Kompositmaterial zur Verfügung gestellt; es weist eine sehr hohe Röntgenabsorption auf. Ein Versuchskomposit mit einem Füllstoffgehalt von ca. 75 Gew.-% besitzt eine Röntgenopazität von ca. 350%.

Für den Fall der Anwendung im Dentalbereich entspricht das Verbundmaterial bezüglich seiner Transluzenz und seiner ästhetischen Wirkung weitestgehend den Werten des natürlichen Zahnmaterials. Es ist im physiologisch-chemischen Sinn stabil und läßt sich mechanisch gut bearbeiten, insbesondere polieren und beschleifen.

In den nachfolgenden Tabellen sind Einzelrezepturen für den röntgenopaken Füllstoff des erfindungsgemäßen Verbundwerkstoffs in Gewichts-Prozenten (Gew.-%) angegeben. In Tabelle 1 sind die Rezepturen von 35 Einzelbeispielen aufgeführt, wobei neben den Komponenten-Gruppen $SiO_2$, $B_2O_3$, $Al_2O_3$ und $Gd_2O_3$, SrO, $La_2O_3$ noch die Oxide der ein- bzw. zweiwertigen Metalle Na, Mg und Ca erscheinen.

In Tabelle 2 sind 10 weitere Rezepturen von Einzelbeispielen angegeben, die neben den beiden essentiellen Komponenten-Gruppen noch Oxide von drei- bis fünfwertigen Metallen aufweisen. Schließlich wurde noch die Brechzahl $n_e$ angegeben.

Tabelle 1 (Gew.-%)

| Bsp.Nr. | 1 | 2 | 3 | 4 | 5 | 6 | 7 | 8 |
|---|---|---|---|---|---|---|---|---|
| $SiO_2$ | 23,66 | 24,00 | 24,36 | 25,18 | 25,30 | 25,28 | 25,07 | 26,71 |
| $B_2O_3$ | 20,38 | 20,68 | 21,00 | 21,10 | 22,00 | 22,01 | 21,82 | 21,47 |
| $Al_2O_3$ | 20,65 | 21,55 | 22,46 | 22,58 | 22,66 | 23,86 | 23,66 | 13,97 |
| $Gd_2O_3$ | 8,16 | 6,21 | 4,20 | 4,23 | 4,24 | 4,24 | 12,62 | 16,56 |
| SrO | 19,82 | 20,12 | 20,42 | 19,32 | 18,18 | 16,98 | 16,83 | 21,29 |
| $La_2O_3$ | 7,33 | 7,44 | 7,56 | 7,59 | 7,62 | 7,63 | – | – |
| $Na_2O$ | – | – | – | – | – | – | – | – |
| MgO | – | – | – | – | – | – | – | – |
| CaO | – | – | – | – | – | – | – | – |
| $n_e$ | 1,585 | 1,580 | 1,576 | 1,576 | 1,572 | 1,569 | 1,568 | 1,587 |

| Bsp.Nr. | 9 | 10 | 11 | 12 | 13 | 14 | 15 | 16 |
|---|---|---|---|---|---|---|---|---|
| $SiO_2$ | 26,94 | 27,87 | 16,30 | 16,18 | 16,81 | 25,97 | 26,15 | 26,09 |
| $B_2O_3$ | 23,24 | 24,88 | 21,95 | 21,77 | 23,40 | 20,37 | 22,57 | 26,32 |
| $Al_2O_3$ | 15,25 | 15,78 | 31,03 | 30,77 | 31,98 | 25,47 | 14,81 | 16,70 |
| $Gd_2O_3$ | 16,69 | 12,96 | 7,88 | 15,64 | 6,09 | 2,21 | 20,26 | 1,14 |
| SrO | 17,88 | 18,51 | 15,76 | 15,64 | 16,25 | 23,99 | 16,21 | 28,72 |
| $La_2O_3$ | – | – | 7,08 | – | 5,47 | 1,99 | – | 1,03 |
| $Na_2O$ | – | – | – | – | – | – | – | – |
| MgO | – | – | – | – | – | – | – | – |
| CaO | – | – | – | – | – | – | – | – |
| $n_e$ | 1,576 | 1,569 | 1,589 | 1,583 | 1,583 | 1,566 | 1,580 | 1,570 |

| Bsp.Nr. | 17 | 18 | 19 | 20 | 21 | 22 | 23 | 24 |
|---|---|---|---|---|---|---|---|---|
| $SiO_2$ | 25,61 | 24,16 | 40,00 | 50,00 | 50,00 | 50,00 | 50,00 | 50,00 |
| $B_2O_3$ | 23,62 | 22,87 | 4,00 | 4,00 | – | – | 4,00 | 5,00 |
| $Al_2O_3$ | 15,62 | 15,12 | 2,00 | 2,00 | 2,00 | 2,00 | 2,00 | 3,00 |
| $Gd_2O_3$ | 23,81 | 26,88 | 5,00 | 5,00 | 5,00 | 5,00 | 5,00 | 5,00 |
| SrO | 11,34 | 10,97 | 21,00 | 20,00 | 20,00 | 20,00 | 20,00 | 20,00 |
| $La_2O_3$ | – | – | 8,00 | 8,00 | 8,00 | 8,00 | 8,00 | 7,00 |
| $Na_2O$ | – | – | 16,00 | – | – | 4,00 | 5,00 | 5,00 |
| MgO | – | – | 4,00 | – | – | – | – | – |
| CaO | – | – | – | 11,00 | 15,00 | 11,00 | 6,00 | 5,00 |
| $n_e$ | 1,580 | 1,590 | 1,585 | 1,601 | 1,618 | 1,606 | 1,591 | 1,589 |

| Bsp.Nr. | 25 | 26 | 27 | 28 | 29 | 30 | 31 | 32 |
|---|---|---|---|---|---|---|---|---|
| $SiO_2$ | 54,08 | 58,00 | 57,00 | 57,00 | 57,00 | 57,00 | 54,00 | 50,00 |
| $B_2O_3$ | 4,08 | 4,00 | 4,00 | 4,00 | 4,00 | 4,00 | 3,50 | 3,50 |
| $Al_2O_3$ | 2,04 | 2,00 | 2,00 | 2,00 | 2,00 | 2,00 | 2,00 | 2,00 |
| $Gd_2O_3$ | 5,10 | 4,00 | 4,00 | 6,00 | 9,00 | 6,00 | 6,00 | 6,00 |
| SrO | 20,41 | 20,00 | 20,00 | 20,00 | 20,00 | 18,00 | 20,00 | 20,00 |
| $La_2O_3$ | 6,13 | 6,00 | 6,00 | 4,00 | 1,00 | 6,00 | 8,00 | 12,00 |
| $Na_2O$ | 4,08 | 4,00 | 5,00 | 5,00 | 5,00 | 5,00 | 5,00 | 5,00 |
| MgO | – | – | – | – | – | – | – | – |
| CaO | 4,08 | 2,00 | 2,00 | 2,00 | 2,00 | 2,00 | 1,50 | 1,50 |
| $n_e$ | 1,576 | 1,558 | 1,560 | 1,560 | 1,558 | 1,559 | 1,573 | 1,589 |

| Bsp.Nr. | 33 | 34 | 35 |
|---|---|---|---|
| $SiO_2$ | 52,00 | 54,00 | 55,00 |
| $B_2O_3$ | 3,50 | 4,00 | 4,00 |
| $Al_2O_3$ | 2,00 | 2,00 | 2,00 |
| $Gd_2O_3$ | 6,00 | 5,00 | 6,00 |
| SrO | 20,00 | 22,00 | 20,00 |
| $La_2O_3$ | 10,00 | 6,00 | 6,00 |
| $Na_2O$ | 5,00 | 5,00 | 5,00 |
| MgO | – | – | – |
| CaO | 1,50 | 2,00 | 2,00 |
| $n_e$ | 1,585 | 1,573 | 1,569 |

Tabelle 2 (Gew.-%)

| Bsp.Nr. | 36 | 37 | 38 | 39 | 40 |
|---|---|---|---|---|---|
| $SiO_2$ | 23,64 | 23,64 | 24,36 | 16,81 | 26,09 |
| $B_2O_3$ | 20,38 | 20,38 | 21,00 | 23,40 | 26,33 |
| $Al_2O_3$ | 20,65 | 20,65 | 22,46 | 31,97 | 16,70 |
| $Gd_2O_3$ | 8,00 | 6,33 | 3,18 | 5,50 | 2,00 |
| SrO | 19,00 | 19,00 | 21,00 | 14,32 | 23,85 |
| $La_2O_3$ | 4,33 | 6,00 | 3,00 | 6,00 | 1,03 |
| $Y_2O_3$ | – | – | – | – | – |
| $TiO_2$ | 4,00 | 4,00 | – | – | – |
| $ZrO_2$ | – | – | – | – | – |
| $Nb_2O_5$ | – | – | – | – | 4,00 |
| $Ta_2O_5$ | – | – | 5,00 | 2,00 | – |

| Bsp.Nr. | 41 | 42 | 43 | 44 | 45 |
|---|---|---|---|---|---|
| $SiO_2$ | 25,28 | 24,16 | 26,15 | 24,36 | 24,36 |
| $B_2O_3$ | 22,01 | 22,87 | 22,57 | 21,00 | 21,00 |
| $Al_2O_3$ | 23,86 | 15,12 | 14,81 | 22,46 | 22,46 |
| $Gd_2O_3$ | 4,00 | 20,88 | 14,47 | 4,20 | 4,20 |
| SrO | 14,85 | 10,97 | 17,00 | 20,42 | 20,42 |
| $La_2O_3$ | 7,50 | 1,00 | 2,50 | 5,06 | 2,56 |
| $Y_2O_3$ | – | 5,00 | 2,50 | – | – |
| $TiO_2$ | – | – | – | – | – |
| $ZrO_2$ | – | – | – | 2,50 | 5,00 |
| $Nb_2O_5$ | 2,50 | – | – | – | – |
| $Ta_2O_5$ | – | – | – | – | – |

Die Löslichkeit dieser anorganischen Füllstoffe ist sehr gering. Nach einer 20-tägigen Behandlung in Wasser bei Temperaturen von 37 °C ergeben sich lediglich Gewichtsverluste von weniger als 0,1%.

Es hat sich überraschend gezeigt, daß der Einsatz einer Gadoliniumverbindung zusammen mit einer Strontiumverbindung bzw. mit einem Gemisch aus einer Sr- und einer La-Verbindung in den angegebenen Grenzen in dem röntgenopaken Füllstoff zu einem überlegenen Gesamtabsorptionsbereich – also zu einer beachtlichen Steigerung der Röntgenopazität des Materials im Vergleich zu den bisherigen anorganischen Röntgenkontrastsubstanzen – geführt hat.

Das SrO kann zur Einstellung der jeweils gewünschten Brechzahl durch die Erdalkalioxide CaO und/oder MgO partiell ersetzt werden. Auch kann die röntgenopazitätsverursachende Dreiergruppe ($Gd_2O_3$ + SrO bzw. $Gd_2O_3$ + SrO + $La_2O_3$) bis zu 5 Gew.-% durch $ZrO_2$ oder innerhalb der erläuterten Grenzen durch weitere Oxide, wie z.B. Titan-, Tantal-, Niob- und/oder Yttrium-Oxid, ersetzt werden. Aus schmelztechnologischen Gründen kann ein Anteil von bis zu 16 Gew.-% $Na_2O$ zugesetzt werden. Dadurch wird die Schmelztemperatur des Gemenges ohne Beeinträchtigung der chemischen Stabilität herabgesetzt.

Ein spezielles Einzelbeispiel eines erfindungsgemäßen Vorprodukts ist wie folgt zusammengesetzt:

75,0 Gew.-% eines röntgenopaken Füllstoffes mit der Zusammensetzung:

54 Gew.-% $SiO_2$
4 Gew.-% $B_2O_3$
2 Gew.-% $Al_2O_3$
5 Gew.-% $Gd_2O_3$
22 Gew.-% SrO
6 Gew.-% $La_2O_3$
5 Gew.-% $Na_2O$
2 Gew.-% CaO
2,0 Gew.-% Aerosil R 972 (hochdisperses $SiO_2$)
16,0 Gew.-% Bis-GMA
6,8 Gew.-% Triäthylenglykoldimethacrylat
0,09 Gew.-% Campherchinon
0,11 Gew.-% N,N-Dimethylaminoäthylmethacrylat.

Die Aushärtung erfolgt unter Einsatz des Lichtgerätes «Translux» mit Tiefenhärtfilter der Firma Kulzer bei einer Bestrahlungszeit von ca. 2 min. Der so erhaltene Verbundwerkstoff weist die folgenden physikalischen Eigenschaften auf:

| | |
|---|---|
| Biegefestigkeit: | 100 MPa |
| Druckfestigkeit: | 270 MPa |
| Transparenz (bei einer Schichtdicke von 3 mm): | 20% |
| Röntgenopazität: | 350% |

**Patentansprüche für die Vertragsstaaten BE, CH, DE, FR, GB, IT, LI**

1. Verbundwerkstoff auf Kunststoffbasis für prothetische Zwecke mit einem röntgenopaken Füllstoff, dadurch gekennzeichnet, daß der röntgenopake Füllstoff Verbindungen der Elemente Gadolinium, Strontium und gegebenenfalls Lanthan als röntgenabsorptionsverursachende Komponenten-Kombination enthält.

2. Verbundwerkstoff nach Anspruch 1, dadurch gekennzeichnet, daß der röntgenopake Füllstoff die Oxid-Gruppe $Gd_2O_3$ + SrO bzw. $Gd_2O_3$ + SrO + $La_2O_3$ enthält.

3. Verbundwerkstoff nach einem der vorhergehenden Ansprüche, dadurch gekennzeichnet, daß der Anteil an röntgenopakem Füllstoff 20,0 bis 90,0 Gew.-% – vorzugsweise 40,0 bis 85,0 Gew.-% beträgt.

4. Verbundwerkstoff nach mindestens einem der vorhergehenden Ansprüche, dadurch gekennzeichnet, daß der röntgenopake Füllstoff aus:
(a) 1 bis 31,0 Gew.-% $Gd_2O_3$
7 bis 32,0 Gew.-% SrO
0 bis 18,0 Gew.-% $La_2O_3$,
wobei die Summe der Oxide $Gd_2O_3$ + SrO + $La_2O_3$ zwischen 24,0 und 40,0 Gew.-% beträgt;
(b) 14 bis 62 Gew.-% $SiO_2$
0 bis 30 Gew.-% $B_2O_3$
1 bis 33 Gew.-% $Al_2O_3$,
wobei die Summe der Oxide $SiO_2$ + $B_2O_3$ + $Al_2O_3$ zwischen 42 und 75 Gew.-% beträgt;
(c) 0 bis 16 Gew.-% $Na_2O$
besteht.

5. Verbundwerkstoff nach Anspruch 4, dadurch gekennzeichnet, daß der SrO-Anteil entweder

(a) bis zu 18 Gew.-% durch CaO oder
(b) bis zu 5 Gew.-% durch MgO ersetzt wird.

6. Verbundwerkstoff nach Anspruch 4, dadurch gekennzeichnet, daß der Anteil an $Gd_2O_3$ und/oder $La_2O_3$ und/oder SrO bis zu 5 Gew.-% durch $ZrO_2$ ersetzt wird.

7. Verbundwerkstoff nach Anspruch 4, dadurch gekennzeichnet, daß der Anteil an $Gd_2O_3$ und/oder $La_2O_3$ und/oder SrO bis zu 5 Gew.-% durch mindestens eines der Oxide: $Y_2O_3$, $TiO_2$, $Ta_2O_5$ und $Nb_2O_5$ ersetzt wird.

8. Verbundwerkstoff nach mindestens einem der Ansprüche 4 bis 6, dadurch gekennzeichnet, daß der röntgenopake Füllstoff aus:
1,0–26,9 Gew.-% $Gd_2O_3$
10,9–28,8 Gew.-% SrO
0–12,0 Gew.-% $La_2O_3$
16,1–58,0 Gew.-% $SiO_2$
0–26,3 Gew.-% $B_2O_3$
2,0–32,0 Gew.-% $Al_2O_3$
0–16,0 Gew.-% $Na_2O$
0–15,0 Gew.-% CaO
0– 4,0 Gew.-% MgO
0– 5,0 Gew.-% $ZrO_2$
0– 5,0 Gew.-% $Y_2O_3$
0– 4,0 Gew.-% $TiO_2$
0– 5,0 Gew.-% $Ta_2O_5$
0– 5,0 Gew.-% $Nb_2O_5$
besteht.

9. Verbundwerkstoff nach mindestens einem der Ansprüche 4 bis 6 und 8, dadurch gekennzeichnet, daß der röntgenopake Füllstoff aus
2,0–10,0 Gew.-% $Gd_2O_3$
15,0–25,0 Gew.-% SrO
1,0–10,0 Gew.-% $La_2O_3$
50,0–60,0 Gew.-% $SiO_2$
2,0– 6,0 Gew.-% $B_2O_3$
1,0– 3,0 Gew.-% $Al_2O_3$
2,0– 8,0 Gew.-% $Na_2O$
1,0– 5,0 Gew.-% CaO
besteht.

10. Verbundwerkstoff nach mindestens einem der Ansprüche 4 bis 9, dadurch gekennzeichnet, daß der röntgenopake Füllstoff als Glas vorliegt und eine Brechzahl $n_e$ zwischen 1,55 und 1,62 aufweist.

11. Verbundwerkstoff nach mindestens einem der Ansprüche 4 bis 9, dadurch gekennzeichnet, daß der röntgenopake Füllstoff als Glaskeramik vorliegt.

12. Verbundwerkstoff nach mindestens einem der Ansprüche 4 bis 9, dadurch gekennzeichnet, daß der röntgenopake Füllstoff als Sinterprodukt vorliegt.

13. Verbundwerkstoff nach mindestens einem der Ansprüche 1 bis 9, dadurch gekennzeichnet, daß der röntgenopake Füllstoff in Form eines Gemisches aus seinen Ausgangskomponenten vorliegt.

14. Verbundwerkstoff nach mindestens einem der vorhergehenden Ansprüche, dadurch gekennzeichnet, daß er zusätzliche Pigmente mit einer Korngröße kleiner als 2 μm enthält.

15. Verbundwerkstoff nach mindestens einem der vorhergehenden Ansprüche, dadurch gekennzeichnet, daß der röntgenopake Füllstoff in Form von silanisierten Partikeln vorliegt.

16. Verbundwerkstoff nach mindestens einem der vorhergehenden Ansprüche, dadurch gekennzeichnet, daß die Partikelgröße des röntgenopaken Füllstoffs 0,5 bis 50 µm beträgt.

17. Verbundwerkstoff nach mindestens einem der vorhergehenden Ansprüche, dadurch gekennzeichnet, daß er zusätzlich bis zu 30 Gew.-% hochdisperses Siliciumdioxid und/oder Aluminiumoxid enthält.

18. Vorprodukt für einen Verbundwerkstoff nach mindestens einem der vorhergehenden Ansprüche, dadurch gekennzeichnet, daß es als organisches Bindemittel mindestens einen Acrylsäureester und/oder einen Methacrylsäureester sowie den röntgenopaken Füllstoff enthält.

19. Vorprodukt nach Anspruch 18, dadurch gekennzeichnet, daß es zusätzlich einen Polymerisationskatalysator enthält.

20. Vorprodukt nach Anspruch 19, dadurch gekennzeichnet, daß der Polymerisationskatalysator ein Photopolymerisationskatalysator ist.

**Patentansprüche für den Vertragsstaat AT**

1. Verfahren zur Herstellung eines Verbundwerkstoffes auf Kunststoffbasis für prothetische Zwecke mit einem röntgenopaken Füllstoff, dadurch gekennzeichnet, daß als röntgenopaker Füllstoff Verbindungen der Elemente Gadolinium, Strontium und gegebenenfalls Lanthan als röntgenabsorptionsverursachende Komponenten-Kombination beigemischt werden.

2. Verfahren nach Anspruch 1, dadurch gekennzeichnet, daß als röntgenopaker Füllstoff die Oxid-Gruppen $Gd_2O_3$ + SrO bzw. $Gd_2O_3$ + SrO + $La_2O_3$ beigemischt wird.

3. Verfahren nach einem der vorhergehenden Ansprüche, dadurch gekennzeichnet, daß an röntgenopakem Füllstoff 20,0 bis 90,0 Gew.-% – vorzugsweise 40,0 bis 85,0 Gew.-% – beigemischt werden.

4. Verfahren nach mindestens einem der vorhergehenden Ansprüche, dadurch gekennzeichnet, daß als röntgenopaker Füllstoff ein Gemisch aus:
a) 1 bis 31,0 Gew.-% $Gd_2O_3$
7 bis 32,0 Gew.-% SrO
0 bis 18,0 Gew.-% $La_2O_3$,
wobei die Summe der Oxide $Gd_2O_3$ + SrO + $La_2O_3$ zwischen 24,0 und 40,0 Gew.-% beträgt:
b) 14 bis 62 Gew.-% $SiO_2$
0 bis 30 Gew.-% $B_2O_3$
1 bis 33 Gew.-% $Al_2O_3$
wobei die Summe der Oxide $SiO_2$ + $B_2O_3$ + $Al_2O_3$ zwischen 42 und 75 Gew.-% beträgt;
c) 0 bis 16 Gew.-% $Na_2O$
zugesetzt wird.

5. Verfahren nach Anspruch 4, dadurch gekennzeichnet, daß der SrO-Anteil entweder
a) bis zu 18 Gew.-% durch CaO oder
b) bis zu 5 Gew.-% durch MgO ersetzt wird.

6. Verfahren nach Anspruch 4, dadurch gekennzeichnet, daß der Anteil an $Gd_2O_3$ und/oder $La_2O_3$ und/oder SrO bis zu 5 Gew.-% durch $ZrO_2$ ersetzt wird.

7. Verfahren nach Anspruch 4, dadurch gekennzeichnet, dass der Anteil an $Gd_2O_3$ und/oder $La_2O_3$ und/oder SrO bis zu 5 Gew.-% durch mindestens eines der Oxide $Y_2O_3$, $TiO_2$, $Ta_2O_5$ und $Nb_2O_5$ ersetzt wird.

8. Verfahren nach mindestens einem der Ansprüche 4 bis 6, dadurch gekennzeichnet, daß als röntgenopaker Füllstoff ein Gemisch aus:

1,0–26,9 Gew.-% $Gd_2O_3$
10,9–28,8 Gew.-% SrO
0–12,0 Gew.-% $La_2O_3$
16,1–58,0 Gew.-% $SiO_2$
0–26,3 Gew.-% $B_2O_3$
2,0–32,0 Gew.-% $Al_2O_3$
0–16,0 Gew.-% $Na_2O$
0–15,0 Gew.-% CaO
0– 4,0 Gew.-% MgO
0– 5,0 Gew.-% $ZrO_2$
0– 5,0 Gew.-% $Y_2O_3$
0– 4,0 Gew.-% $TiO_2$
0– 5,0 Gew.-% $Ta_2O_5$
0– 5,0 Gew.-% $Nb_2O_5$
zugesetzt wird.

9. Verfahren nach mindestens einem der Ansprüche 4 bis 6 und 8, dadurch gekennzeichnet, daß als röntgenopaker Füllstoff ein Gemisch aus
2,0–10,0 Gew.-% $Gd_2O_3$
15,0–25,0 Gew.-% SrO
1,0–10,0 Gew.-% $La_2O_3$
50,0–60,0 Gew.-% $SiO_2$
2,0– 6,0 Gew.-% $B_2O_3$
1,0– 3,0 Gew.-% $Al_2O_3$
2,0– 8,0 Gew.-% $Na_2O$
1,0– 5,0 Gew.-% CaO
zugesetzt wird.

10. Verfahren nach mindestens einem der Ansprüche 4 bis 9, dadurch gekennzeichnet, daß der röntgenopake Füllstoff als Glas gefertigt wird, das eine Brechzahl $n_e$ zwischen 1,55 und 1,62 aufweist.

11. Verfahren nach mindestens einem der Ansprüche 4 bis 9, dadurch gekennzeichnet, daß der röntgenopake Füllstoff als Glaskeramik gefertigt wird.

12. Verfahren nach mindestens einem der Ansprüche 4 bis 9, dadurch gekennzeichnet, daß der röntgenopake Füllstoff gesintert wird.

13. Verfahren nach mindestens einem der Ansprüche 1 bis 9, dadurch gekennzeichnet, daß der röntgenopake Füllstoff durch Bereitung eines Gemisches aus seinen Ausgangskomponenten hergestellt wird.

14. Verfahren nach mindestens einem der vorhergehenden Ansprüche, dadurch gekennzeichnet, daß dem Verbundwerkstoff zusätzliche Pigmente mit einer Korngrösse kleiner als 2 µm zugesetzt werden.

15. Verfahren nach mindestens einem der vorhergehenden Ansprüche, dadurch gekennzeichnet, daß der röntgenopake Füllstoff als silanisierte Partikel zugemischt wird.

16. Verfahren nach mindestens einem der vorhergehenden Ansprüche, dadurch gekennzeichnet, daß der röntgenopake Füllstoff mit einer Partikelgröße von 0,5 bis 50 µm eingesetzt wird.

17. Verfahren nach mindestens einem der vorhergehenden Ansprüche, dadurch gekennzeichnet, daß dem Verbundwerkstoff zusätzlich bis zu 30 Gew.-% hochdisperses Siliciumdioxid und/oder Aluminiumoxid zugemischt werden.

18. Verfahren zur Herstellung eines Vorproduktes für einen nach mindestens einem der vorhergehenden Ansprüche hergestellten Verbundwerkstoff, dadurch gekennzeichnet, daß für das Vorprodukt als organisches Bindemittel mindestens ein Acrylsäureester und/oder ein Methacrylsäureester sowie der röntgenopake Füllstoff gemischt werden.

19. Verfahren nach Anspruch 18, dadurch gekennzeichnet, daß dem Vorprodukt zusätzlich ein Polymerisationskatalysator zugesetzt wird.

20. Verfahren nach Anspruch 19, dadurch gekennzeichnet, daß als Polymerisationskatalysator ein Photopolymerisationskatalysator eingesetzt wird.

**Claims**

1. Compound material on a synthetic material base for prothetic purposes with a filler opaque to X-rays, characterised thereby, that the filler opaque to X-rays contains compounds of the elements gadolinium, strontium and, in a given case, lanthanum as component combination causing absorption of X-rays.

2. Compound material according to claim 2, characterised thereby, that the filler opaque to X-rays contains the oxide group of $Gd_2O_3$ + SrO or $Gd_2O_3$ + SrO + $La_2O_3$.

3. Compound material according to one of the preceding claims, characterised thereby, that the proportion of filler opaque to X-rays amounts to 20.0 to 90.0% by weight, preferably 40.0 to 85.0% by weight.

4. Compound material according to at least one of the preceding claims, characterised thereby, that the filler opaque to X-rays consists of
(a) 1 to 31.0% by weight of $Gd_2O_3$
7 to 32.0% by weight of SrO and
0 to 18.0% by weight of $La_2O_3$,
wherein the sum of the oxides $Gd_2O_3$ + SrO + $La_2O_3$ amounts to between 24.0 and 40% by weight;
(b) 14 to 62% by weight of $SiO_2$
0 to 30% by weight of $B_2O_3$ and
1 to 33% by weight of $Al_2O_3$,
wherein the sum of the oxides $SiO_2$ + $B_2O_3$ + $Al_2O_3$ amounts to between 42 and 75% by weight;
(c) 0 to 16% by weight of $Na_2O$.

5. Compound material according to claim 4, characterised thereby, that the proportion of SrO is substituted by either
(a) up to 18% by weight of CaO or
(b) up to 5% by weight of MgO.

6. Compound material according to claim 4, characterised thereby, that the proportion of $Gd_2O_3$ and/or $La_2O_3$ and/or SrO is substituted by up to 5% by weight of $ZrO_2$.

7. Compound material according to claim 4, characterised thereby, that the proportion of $Gd_2O_3$ and/or $La_2O_3$ and/or SrO is substituted by up to 5% by weight of at least one of the oxides $Y_2O_3$, $TiO_2$, $Ta_2O_5$ and $Nb_2O_5$.

8. Compound material according to at least one of the claims 4 to 6, characterised thereby, that the filler opaque to X-rays consists of
1.0 to 26.9% by weight of $Gd_2O_3$
10.9 to 28.8% by weight of SrO
0 to 12.0% by weight of $La_2O_3$
16.1 to 58.0% by weight of $SiO_2$
0 to 26.3% by weight of $B_2O_3$
2.0 to 32.0% by weight of $Al_2O_3$
0 to 16.0% by weight of $Na_2O$
0 to 15.0% by weight of CaO
0 to 4.0% by weight of MgO
0 to 5.0% by weight of $ZrO_2$
0 to 5.0% by weight of $Y_2O_3$
0 to 4.0% by weight of $TiO_2$
0 to 5.0% by weight of $Ta_2O_5$ and
0 to 5.0% by weight of $Nb_2O_5$.

9. Compound material according to at least one of the claims 4 to 6 and 8, characterised thereby, that the filler opaque to X-rays consists of
2.0 to 10.0% by weight of $Gd_2O_3$
15.0 to 25.0% by weight of SrO
1.0 to 10.0% by weight of $La_2O_3$
50.0 to 60.0% by weight of $SiO_2$
2.0 to 6.0% by weight of $B_2O_3$
1.0 to 3.0% by weight of $Al_2O_3$
2.0 to 8.0% by weight of $Na_2O$ and
1.0 to 5.0% by weight of CaO.

10. Compound material according to at least one of the claims 4 to 9, characterised thereby, that the filler opaque to X-rays is present as glass and displays a refractive index $n_e$ of between 1.55 and 1.62.

11. Compound material according to at least one of the claims 4 to 9, characterised thereby, that the filler opaque to X-rays is present as glass-ceramic.

12. Compound material according to at least one of the claims 4 to 9, characterised thereby, that the filler opaque to X-rays is present as sintered product.

13. Compound material according to at least one of the claims 1 to 9, characterised thereby, that the filler opaque to X-rays is present in the form of a mixture of its starting components.

14. Compound material according to at least one of the preceding claims, characterised thereby, that it contains additional pigments of a grain size of less than 2 micrometres.

15. Compound material according to at least one of the preceding claims, characterised thereby, that the filler opaque to X-rays is present in the form of silanised particles.

16. Compound material according to at least one of the preceding claims, characterised thereby, that the particle size of the filler opaque to X-rays amounts to 0.5 to 50 micrometres.

17. Compound material according to at least one of the preceding claims, characterised thereby, that it contains additionally up to 30% by weight of highly dispersed silicon oxide and/or aluminium oxide.

18. Initial product for a compound material according to at least one of the preceding claims, characterised thereby, that it contains at least one acrylate and/or one methacrylate as well as the filler opaque to X-rays as organic binding agent.

19. Initial product according to claim 18, characterised thereby, that it contains a polymerisation catalyst in addition.

20. Initial product according to claim 19, characterised thereby, that the polymerisation catalyst is a photopolymerisation catalyst.


## Revendications

1. Matériau composite à base de matière synthétique pour prothèses avec une charge opaque aux rayons X, caractérisé en ce que la charge opaque aux rayons X contient des composés des éléments gadolinium, strontium et éventuellement lanthane en tant que combinaison de constituants causant une absorption des rayons X.

2. Matériau composite selon la revendication 1, caractérisé en ce que la charge opaque aux rayons X contient le groupe d'oxydes $Gd_2O_3$ + SrO ou $Gd_2O_3$ + SrO + $La_2O_3$.

3. Matériau composite selon l'une des revendications précédentes, caractérisé en ce que la fraction de charge opaque aux rayons X représente 20,0 à 90,0% en poids – de préférence 40,0 à 85,0% en poids.

4. Matériau composite selon l'une au moins des revendications précédentes, caractérisé en ce que la charge opaque aux rayons X consiste en:
(a) 1 à 31,0% en poids de $Gd_2O_3$
7 à 32,0% en poids de SrO
0 à 18,0% en poids de $La_2O_3$,
la somme des oxydes $Gd_2O_3$ + SrO + $La_2O_3$ étant comprise entre 24,0 et 40,0% en poids;
(b) 14 à 62% en poids de $SiO_2$
0 à 30% en poids de $B_2O_3$
1 à 33% en poids de $Al_2O_3$,
la somme des oxydes $SiO_2$ + $B_2O_3$ + $Al_2O_3$ étant comprise entre 42 et 75% en poids;
(c) 0 à 16% en poids de $Na_2O$.

5. Matériau composite selon la revendication 4, caractérisé en ce que la fraction de SrO est remplacée soit
(a) à raison de jusqu'à 18% en poids par CaO, ou
(b) à raison de jusqu'à 5% en poids par MgO.

6. Matériau composite selon la revendication 4, caractérisé en ce que la fraction de $Gd_2O_3$ et/ou de $La_2O_3$ et/ou de SrO est remplacée à raison de jusqu'à 5% en poids par $ZrO_2$.

7. Matériau composite selon la revendication 4, caractérisé en ce que la fraction de $Gd_2O_3$ et/ou de $La_2O_3$ et/ou de SrO est remplacée à raison de jusqu'à 5% en poids par au moins l'un des oxydes: $Y_2O_3$, $TiO_2$, $Ta_2O_5$ et $Nb_2O_5$.

8. Matériau composite selon l'une au moins des revendications 4 à 6, caractérisé en ce que la charge opaque aux rayons X consiste en:
1,0–26,9% en poids de $Gd_2O_3$
10,9–28,8% en poids de SrO
0–12,0% en poids de $La_2O_3$
16,1–58,0% en poids de $SiO_2$
0–26,3% en poids de $B_2O_3$
2,0–32,0% en poids de $Al_2O_3$
0–16,0% en poids de $Na_2O$
0–15,0% en poids de CaO
0– 4,0% en poids de MgO
0– 5,0% en poids de $ZrO_2$
0– 5,0% en poids de $Y_2O_3$
0– 4,0% en poids de $TiO_2$
0– 5,0% en poids de $Ta_2O_5$
0– 5,0% en poids de $Nb_2O_5$

9. Matériau composite selon l'une au moins des revendications 4 à 6 et 8, caractérisé en ce que la charge opaque aux rayons X consiste en:
2,0–10,0% en poids de $Gd_2O_3$
15,0–25,0% en poids de SrO
1,0–10,0% en poids de $La_2O_3$
50,0–60,0% en poids de $SiO_2$
2,0– 6,0% en poids de $B_2O_3$
1,0– 3,0% en poids de $Al_2O_3$
2,0– 8,0% en poids de $Na_2O$
1,0– 5,0% en poids de CaO.

10. Matériau composite selon l'une au moins des revendications 4 à 9, caractérisé en ce que la charge opaque aux rayons X est sous forme de verre et présente un indice de réfraction $n_e$ compris entre 1,55 et 1,62.

11. Matériau composite selon l'une au moins des revendications 4 à 9, caractérisé en ce que la charge opaque aux rayons X est sous forme d'une vitrocéramique.

12. Matériau composite selon au moins l'une des revendications 4 à 9, caractérisé en ce que la charge opaque aux rayons X est sous forme d'un produit fritté.

13. Matériau composite selon l'une au moins des revendications 1 à 9, caractérisé en ce que la charge opaque aux rayons X est sous forme d'un mélange de ses constituants de départ.

14. Matériau composite selon l'une au moins des revendications précédentes, caractérisé en ce qu'il contient des pigments supplémentaires avec une taille de grains inférieure à 2 μm.

15. Matériau composite selon l'une au moins des revendications précédentes, caractérisé en ce que la charge opaque aux rayons X est sous forme de particules silanisées.

16. Matériau composite selon l'une au moins des revendications précédentes, caractérisé en ce que la taille des particules de la charge opaque aux rayons X est comprise entre 0,5 et 50 μm.

17. Matériau composite selon l'une au moins des revendications précédentes, caractérisé en ce qu'il contient en outre jusqu'à 30% en poids d'oxyde de silicium et/ou d'oxyde d'aluminium hautement dispersé.

18. Précurseur d'un matériau composite selon l'une au moins des revendications précédentes, caractérisé en ce qu'il contient en tant que liant organique au moins un ester acrylique et/ou un ester méthacrylique ainsi que la charge opaque aux rayons X.

19. Précurseur selon la revendication 18, caractérisé en ce qu'il contient en outre un catalyseur de polymérisation.

20. Précurseur selon la revendication 19, caractérisé en ce que le catalyseur de polymérisation est un catalyseur de photopolymérisation.